# EUROPEAN PATENT APPLICATION

(11) **EP 1 495 720 A1**
(43) Date of publication of application: **12.01.2005**
(21) Application number: 04254054.2
(22) Date of filing: 07.07.2004
(51) Int. Cl.: A61B 6/03

(54) **X-ray computerised tomography (CT) imaging method and system**

(30) Priority: 07.07.2003 JP 2003192894; 11.05.2004 JP 2004141198
(71) Applicant: GE Medical Systems Global Technology Company LLC, Waukesha, Wisconsin 53188-1696 (US)
(72) Inventor: Nishide, Akihiko, Hino-shi Tokyo 191-8503 (JP); Horiuchi, Tetsuya, Hino-shi Tokyo 191-8503 (JP); Hagiwara, Akira, Hino-shi Tokyo 191-8503 (JP)
(74) Representative: Pedder, James Cuthbert

(57) **Abstract**

An object of the present invention is to utilize a distance, which is linearly moved for acceleration or deceleration, out of an overall distance linearly moved during a helical scan for the purpose of image reconstruction. Projection data is acquired even during acceleration or deceleration of linear movement made for a helical scan (A1-A12). The acquired projection data is utilized for image reconstruction. Moreover, during the acceleration of linear movement, while a tube current is being increased (A4), projection data is acquired (A5). During the deceleration of linear movement, while the tube current is being decreased (A9), projection data is acquired (A10).

## Description

The present invention relates to an X-ray computed tomography (CT) method and an X-ray CT system. More particularly, the present invention relates to an X-ray CT imaging method and an X-ray CT system that can utilize a distance, which is moved linearly for acceleration or deceleration, out of an overall distance moved linearly by a table during a helical scan for the purpose of image reconstruction.

For a helical scan, an X-ray tube and an X-ray detector are rotated about a subject of radiography, and a table on which the subject of radiography lies down is moved linearly. In the linear movement, the table that stands still is accelerated up to a predetermined velocity. When the table enters a zone in which projection data should be acquired, the table is retained at the predetermined velocity. After the acquisition of projection data is completed, the table is decelerated to stand still. The predetermined velocity may be set to different values depending on a region to be radiographed. For example, for a certain region to be radiographed, the predetermined speed is set to a velocity V1. For other region to be radiographed, the predetermined speed is set to a velocity V2 (Japanese Unexamined Patent Publication No. 10(1998)-314162 ([0049] to [0051], Fig. 5))

In the past, projection data to be used to reconstruct images is acquired while a linearly moving velocity is held constant but not acquired while linear movement is accelerated or decelerated.

In other words, in conventional X-ray CT systems, a distance moved linearly for acceleration or deceleration out of an overall distance moved linearly is not utilized for image reconstruction but is wasted.

Therefore, an object of the present invention is to provide an X-ray CT imaging method and an X-ray CT system making it possible to utilize a distance, which is moved linearly for acceleration or deceleration, out of an overall distance moved linearly for the purpose of image reconstruction.

According to the first aspect of the present invention, there is provided an X-ray CT imaging method making it possible to acquire projection data even when the linear movement of a table is accelerated or decelerated during a helical scan, and to utilize the acquired projection data for image reconstruction.

In the X-ray CT imaging method in accordance with the first aspect, not only when a linearly moving velocity is held constant but also when linear movement is accelerated or decelerated, projection data is acquired and the acquired projection data is utilized for image reconstruction. Consequently, a distance moved linearly for acceleration or deceleration out of an overall distance moved linearly can be utilized for image reconstruction.

Incidentally, the image reconstruction may be achieved according to a two-dimensional image reconstruction technique or a three-dimensional image reconstruction technique.

According to the second aspect of the present invention, there is provided an X-ray CT imaging method of: acquiring projection data even when the linear movement of a table is accelerated or decelerated during a helical scan; appending coordinate information, which represents the position of the table in a body-axis (hereinafter z-axis) direction during the scan, to each view or several views or holding the coordinate information as separate information; and utilizing the acquired projection data for image reconstruction together with the z-coordinate information synchronous with each view or every several views.

In the X-ray CT imaging method according to the second aspect, projection data is acquired not only when a linearly moving velocity is held constant but also linear movement is accelerated or decelerated. The acquired projection data is utilized for image reconstruction together with z-axis coordinate information. Consequently, a distance moved linearly for acceleration or deceleration within an overall distance moved linearly by the table can be utilized for image reconstruction.

Incidentally, the image reconstruction may refer to two-dimensional image reconstruction or three-dimensional image reconstruction.

According to the third aspect of the present invention, there is provided an X-ray CT imaging method in which image reconstruction is performed concurrently with acquisition of projection data.

In the X-ray CT imaging method according to the third aspect, since image reconstruction is performed concurrently with acquisition of projection data, a time lag spent until images are produced can be minimized.

According to the fourth aspect of the present invention, there is provided an X-ray CT imaging method in which parameters based on which a certain view of projection data is used for image reconstruction are predicted and preserved prior to acquisition of the projection data, or projection data is acquired during prediction of the parameters.

In the X-ray CT imaging method according to the fourth aspect, parameters based on which a certain view of projection data is used for image reconstruction are preserved prior to acquisition of the projection data. Therefore, after the projection data is acquired, image reconstruction can be resumed immediately.

According to the fifth aspect of the present invention, there is provided an X-ray CT imaging method in which: linear movement information representing a change in the position of the table is preserved in advance; a z-coordinate representing the position of the table at which a certain view of projection data is acquired is inferred from the linear movement information prior to acquisition of the projection data; and parameters based on which the projection data is used for image reconstruction are calculated based on the inferred z-coordinate.

In the X-ray CT imaging method according to the fifth aspect, linear movement information representing a change in the position of the table is preserved. Parameters based on which a certain view of projection data is used for image reconstruction are calculated prior to acquisition of the certain view of projection data. Therefore, as soon as projection data is acquired, image reconstruction can be resumed.

According to the sixth aspect of the present invention, there is provided an X-ray CT imaging method different from the foregoing X-ray CT imaging methods in a point that: when the linear movement of a table is accelerated, while a tube current is being increased, projection data is acquired; and when the linear movement is decelerated, while the tube current is being decreased, projection data is acquired.

During acceleration, a distance moved linearly per unit time gets gradually longer. Therefore, if the tube current is held constant, an X-ray density in the direction of linear movement gradually diminishes. On the other hand, during deceleration, the distance moved linearly per unit time gets gradually shorter. Therefore, if the tube current is held constant, the X-ray density in the direction of linear movements gradually increases. In short, a substantial tube current associated with acquired projection data varies depending on a view. This makes preprocessing complicated.

Consequently, in the X-ray CT imaging method in accordance with the sixth aspect, when linear movement is accelerated, while the tube current is being increased, projection data is acquired. When the linear movement is decelerated, while the tube current is being decreased, projection data is acquired. This makes the X-ray density in the direction of linear movement constant. In short, the substantial tube current associated with the projection data acquired during the acceleration or deceleration of linear movement can be held constant irrespective of a view. This leads to simplified preprocessing.

According to the seventh aspect of the present invention, there is provided an X-ray CT imaging method different from the aforesaid X-ray CT imaging methods in a point that linear movement is accelerated or decelerated linearly to a time.

In the X-ray CT imaging method in accordance with the seventh aspect, linear movement is accelerated or decelerated linearly to a time. It is therefore easy to control the acceleration or deceleration.

According to the eighth aspect of the present invention, there is provided an X-ray CT imaging method different from the aforesaid X-ray CT imaging methods in a point that linear movement is accelerated or decelerated nonlinearly to a time.

In the X-ray CT imaging method in accordance with the eighth aspect, linear movement is accelerated or decelerated nonlinearly to a time. Consequently, a change in a linearly moving velocity can be smoothed.

According to the ninth aspect of the present invention, there is provided an X-ray CT imaging method different from the aforesaid X-ray CT imaging methods in a point that projection data is acquired using a multi-detector.

In the X-ray CT imaging method in accordance with the ninth aspect, lots of projection data items can be acquired at a time owing to the multi-detector.

According to the tenth aspect of the present invention, there is provided an X-ray CT imaging method different from the aforesaid X-ray CT imaging methods in a point described below. Namely, assume that an xy plane parallel to an x axis and a y axis is regarded as an image reconstruction plane and that a z-axis direction is regarded as a direction in which arrays of detectors constituting the multi-detector is lined. In this case, based on a distance from the xy plane, which passes the center in the z-axis direction of the multi-detector set at a certain position in order to acquire a view, to the image reconstruction plane and the position of a pixel in the image reconstruction plane, projection data to be used to calculate the pixel value of the pixel is sampled from the view.

Conventional image reconstruction methods are formulated on the assumption that a linearly moving velocity is held constant. Therefore, when a conventional image reconstruction method is adapted to projection data, which is acquired during acceleration or deceleration of linear movement, as it is, an artifact occurs.

In the X-ray CT imaging method in accordance with the tenth aspect, based on the distance in the z-axis direction from the xy plane, which passes the center in the z-axis direction of the multi-detector located at a certain position in order to acquire a view, to the image reconstruction plane, and the position of a pixel g in the image reconstruction plane, projection data to be used to calculate the pixel value of the pixel g is sampled from the view. Consequently, required projection data can be sampled from even projection data items acquired during acceleration or deceleration of linear movement. An artifact can be prevented.

According to the eleventh aspect of the present invention, there is provided an X-ray CT imaging method different from the aforesaid X-ray CT imaging methods in a point that image reconstruction is achieved according to a three-dimensional image reconstruction method.

In the X-ray CT imaging method in accordance with the eleventh aspect, the multi-detector capable of receiving a conical beam spreading at a large angle is used to acquire projection data. Since the three-dimensional image reconstruction technique is adopted for image reconstruction, an artifact attributable to the large angle of the conical beam can be prevented.

Incidentally, the three-dimensional image reconstruction technique includes the Feldkamp technique and weighted Feldkamp technique.

According to the twelfth aspect of the present invention, there is provided an X-ray CT imaging method different from the aforesaid X-ray CT imaging methods in a point described below. Namely, the three-dimensional image reconstruction technique comprises the steps of: arranging acquired projection data items based on positions in the z-axis direction at which the projection data items constituting each view are acquired; sampling projection data items representing one line in a field of view or a plurality of parallel lines adjoining ones of which are separated from each other with a plurality of pixels between them; multiplying projection data items representing each line by conical beam reconstruction weights in order to produce projection line data items; filtering the projection line data items in order to produce image point line data items; calculating back projection pixel data representing each pixel in the field of view based on each image point line data; and adding up back projection pixel data items calculated from all views used to reconstruct images relative to each pixel in order to produce back projection data.

In the X-ray CT imaging method in accordance with the twelfth aspect, the three-dimensional image reconstruction techniques proposed in Patent Applications Nos. 2002-147231 and 2002-238947 can be adopted. Consequently, the number of arithmetic operations can be reduced largely.

According to the thirteenth aspect of the present invention, there is provided an X-ray CT system comprising: an X-ray tube; an X-ray detector; a scanning means that rotates at least one of the X-ray tube and X-ray detector about a subject of radiography, moves both the X-ray tube and X-ray detector relatively to each other and linearly to the subject of radiography, and acquires projection data even during acceleration or deceleration of linear movement; and an image reconstruction means that produces CT images on the basis of acquired projection data.

The X-ray CT imaging method in accordance with the thirteenth aspect is adapted to the X-ray CT system in accordance with the tenth aspect.

According to the fourteenth aspect of the present invention, there is provided an X-ray CT system comprising: an X-ray tube; an X-ray detector; a scanning means for rotating at least one of the X-ray tube and X-ray detector about a subject of radiography, moving of them relatively linearly to the subject of radiography, acquiring projection data even during acceleration or deceleration of linear movement, and appending coordinate information, which represents the position of a table in a body-axis (hereinafter z-axis) direction during a scan, to each view or several views, or preserving the coordinate information as separate information; and an image reconstruction means for producing CT images on the basis of the acquired projection data and the z-coordinate information synchronous with each view or every several views.

The X-ray CT imaging method in accordance with the second aspect can be adapted to the X-ray CT system according to the fourteenth aspect.

According to the fifteenth aspect of the present invention, there is provided an X-ray CT system different from the above X-ray CT system in which image reconstruction executed by the image reconstruction means is performed concurrently with acquisition of projection data executed by the scanning means.

The X-ray CT imaging method in accordance with the third aspect can be adapted to the X-ray CT system in accordance with the fifteenth aspect.

According to the sixteenth aspect of the present invention, there is provided an X-ray CT system further comprising a parameter preserving means for predicting and preserving parameters, based on which a certain view of projection data is used for image reconstruction, prior to acquisition of the projection data, or for predicting and preserving the parameters during acquisition of the projection data.

The X-ray CT imaging method in accordance with the fourth aspect can be adapted to the X-ray CT system in accordance with the sixteenth aspect.

According to the seventeenth aspect of the present invention, there is provided an X-ray CT system further comprising a linear movement information preserving means for preserving in advance linear movement information representing a change in the position of the table caused by the linear movement, and a parameter inferring means that infers a z-coordinate, which represents the position of the table at which a certain view of projection data is acquired, from the linear movement information prior to acquisition of the projection data, and calculates parameters, based on which the projection data is used for image reconstruction, according to the inferred z-coordinate.

The X-ray CT imaging method in accordance with the fifth aspect can be adapted to the X-ray CT system in accordance with the seventeenth aspect.

According to the eighteenth aspect of the present invention, there is provided an X-ray CT system different from the foregoing X-ray CT systems in a point that the scanning means acquires projection data while increasing a tube current during acceleration of linear movement, or acquires projection data while decreasing the tube current during deceleration of linear movement.

The X-ray CT imaging method in accordance with the eithteenth aspect can be adapted to the X-ray CT system in accordance with the twelfth aspect.

According to the nineteenth aspect of the present invention, there is provided an X-ray CT system different from the aforesaid X-ray CT systems in a point that the scanning means accelerates or decelerates linear movement linearly to a time.

The X-ray CT imaging method in accordance with the nineteenth aspect can be adapted to the X-ray CT system in accordance with the thirteenth aspect.

According to the twentyth aspect of the present invention, there is provided an X-ray CT system different from the aforesaid X-ray CT systems in a point that the scanning means accelerates or decelerates linear movement nonlinearly to a time.

The X-ray CT imaging method in accordance with the twentyth aspect can be adapted to the X-ray CT system in accordance with the fourteenth aspect.

According to the twenty-first aspect of the present invention, there is provided an X-ray CT system different from the aforesaid X-ray CT systems in a point that the X-ray detector is a multi-detector.

The X-ray CT imaging method in accordance with the twenty-first aspect can be adapted to the X-ray CT system in accordance with the fifteenth aspect.

According to the twenty-second aspect of the present invention, there is provided an X-ray CT system different from the aforesaid X-ray CT systems in a point described below. Namely, assume that an xy plane parallel to an x axis and a y axis is regarded as an image reconstruction plane, and that a z-axis direction is regarded as a direction in which arrays of detectors constituting a multi-detector are lined. In this case, based on a distance in the z-axis direction from the xy plane, which passes the center in the z-axis direction of the multi-detector located at a certain position in order to acquire a view, to the image reconstruction plane, and the position of a pixel in the image reconstruction plane, the image reconstruction means samples projection data to be used to calculate the pixel value of the pixel from the view.

The X-ray CT imaging method in accordance with the twenty-second aspect can be adapted to the X-ray CT system in accordance with the sixteenth aspect.

According to the twenty-third aspect of the present invention, there is provided an X-ray CT system different from the aforesaid X-ray CT systems in a point that the image reconstruction means performs image reconstruction according to a three-dimensional image reconstruction technique.

The X-ray CT imaging method in accordance with the twenty-third aspect can be adapted to the X-ray CT system in accordance with the seventeenth aspect.

According to the twenty-fourth aspect of the present invention, there is provided an X-ray CT system different from the aforesaid X-ray CT systems in a point described below. Namely, the three-dimensional image reconstruction technique comprises the steps of: arranging acquired projection data items based on positions in the z-axis direction at which the projection data items constituting each view are acquired; sampling projection data items representing one line in a field of view or a plurality of parallel lines adjoining ones of which are separated from each other with a plurality of pixels between them; multiplying projection data items representing each line by conical beam reconstruction weights in order to produce projection line data items; filtering the projection line data items in order to produce image point line data items; calculating back projection pixel data representing each pixel in the field of view based on each image point line data; and adding up back projection pixel data items calculated from all views to be used to reconstruct images relative to each pixel in order to produce back projection data.

The X-ray CT imaging method in accordance with the twenty-fourth aspect can be adapted to the X-ray CT system in accordance with the eighteenth aspect.

According to an X-ray CT imaging method and X-ray CT system in which the present invention is implemented, a distance linearly moved for acceleration or deceleration out of an overall distance linearly moved during a helical scan can be utilized for image reconstruction.

The X-ray CT imaging method and X-ray CT system in accordance with the present invention can be utilized for production of X-ray CT images.

The invention will now be described in greater detail, by way of example, with reference to the drawings, in which:-
Fig. 1 is a block diagram showing an X-ray CT system in accordance with the first embodiment of the present invention.
Fig. 2 is an explanatory view showing rotation of an X-ray tube and a multi-detector.
Fig. 3 is an explanatory diagram showing a conical beam.
Fig. 4 is a flowchart outlining actions to be performed in the X-ray CT system in accordance with the first embodiment of the present invention.
Fig. 5 is a flowchart describing data acquisition.
Fig. 6 is a flowchart describing data acquisition.
Fig. 7 is a graph indicating a change in a linearly moving velocity occurring when a cradle is linearly accelerated or decelerated.
Fig. 8 is a graph indicating a change in a tube current occurring when the cradle is linearly accelerated or decelerated.
Fig. 9 is a graph indicating a change in the linearly moving velocity occurring when the cradle is nonlinearly accelerated or decelerated.
Fig. 10 is a graph indicating a change in the tube current occurring when the cradle is nonlinearly accelerated or decelerated.
Fig. 11 is a graph indicating a change in the linearly moving velocity occurring when the cradle is linearly accelerated or decelerated without moved at a constant velocity.
Fig. 12 is a graph indicating a change in the tube current occurring when the cradle is linearly accelerated or decelerated without moved at a constant velocity.
Fig. 13 is a graph indicating a change in the linearly moving velocity occurring when the cradle is nonlinearly accelerated or decelerated without moved at a constant velocity.
Fig. 14 is a graph indicating a change in the tube current occurring when the cradle is nonlinearly accelerated or decelerated without moved at a constant velocity.
Fig. 15 is a flowchart describing three-dimensional image reconstruction.
Fig. 16 is a conceptual diagram showing projection of lines in a field of view in the direction in which X-rays are transmitted.
Fig. 17 is a conceptual diagram showing lines projected on the surface of a detector.
Fig. 18 is a conceptual diagram showing development of projection data items Dr, which represent each of lines and are produced with an X-ray tube set at a view angle 0°, on a plane of projection.
Fig. 19 is a conceptual diagram showing development of projection line data items Dp, which represent each of the lines and are produced with the X-ray tube set at the view angle 0°, on the plane of projection.
Fig. 20 is a conceptual diagram showing development of high-density image point line data items Df, which represent each of the lines and are produced with the X-ray tube set at the view angle 0°, on the plane of projection.
Fig. 21 is a conceptual diagram showing back projection pixel data items D2 that represent each of the lines and that are produced with the X-ray tube set at the view angle 0°.
Fig. 22 is a conceptual diagram showing the back projection pixel data items D2 that represent the pixels in the field of view and that are produced with the X-ray tube set at the view angle 0°.
Fig. 23 is a conceptual diagram showing development of projection data items Dr, which represent each of lines and are produced with the X-ray tube set at a view angle 90°, on the plane of projection.
Fig. 24 is a conceptual diagram showing development of projection line data items Dp, which represent each of the lines and are produced with the X-ray tube set at the view angle 90°, on the plane of projection.
Fig. 25 is a conceptual diagram showing development of high-density image point line data items Dh, which represent each of the lines and are produced with the X-ray tube set at the view angle 90°, on the plane of projection.
Fig. 26 is a conceptual diagram showing back projection pixel data items D2 that represent each of the lines in the field of view and that are produced with the X-ray tube set at the view angle 90°.
Fig. 27 is a conceptual diagram showing back projection pixel data items D2 that represent the pixels in the field of view and that are produced with the X-ray tube set at the view angle 90°.
Fig. 28 is an explanatory diagram showing a process of calculating back projection data D3 by adding up the back projection pixel data items D2, which are produced from all views, in relation to each pixel.
Fig. 29 is a flowchart describing data acquisition to be executed according to a second embodiment.
Fig. 30 is a flowchart describing parameter inference to be executed according to the second embodiment.
Fig. 31 is a flowchart describing three-dimensional back projection to be executed according to the second embodiment.

The present invention will be described by taking an illustrated embodiment for instance. Noted is that the present invention is not limited to the embodiment.

### [First Embodiment]

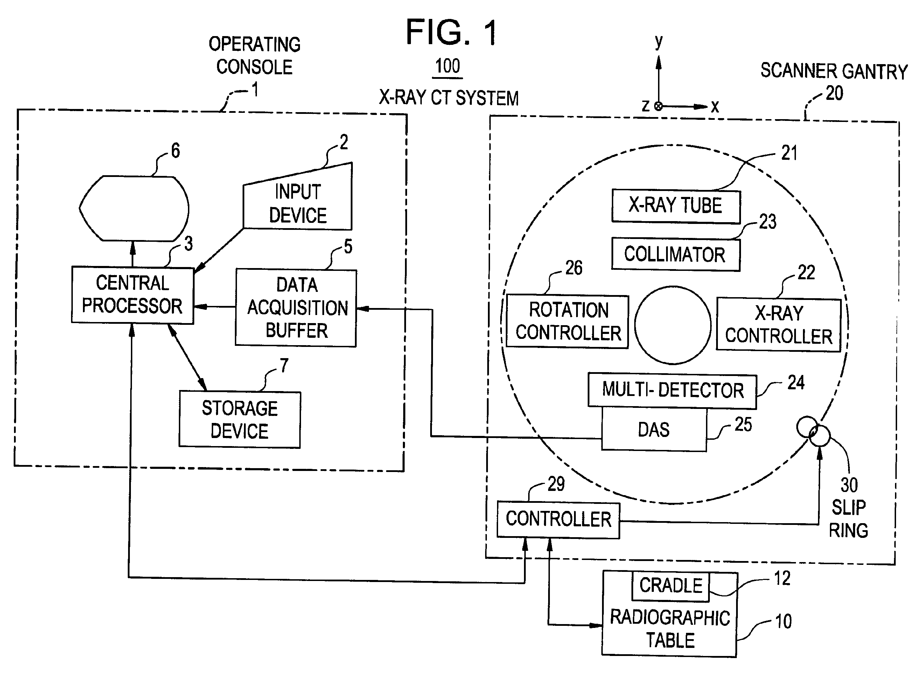

Fig. 1 is a block diagram showing the configuration of an X-ray CT system in accordance with an embodiment of the present invention.

The X-ray CT system 100 comprises an operating console 1, a radiographic table 10, and a scanner gantry 20.

The operating console 1 comprises: an input device 2 that receives an operator's entry; a central processor 3 that executes image reconstruction or the like; a data acquisition buffer 5 in which projection data acquired by the scanner gantry 20 is held; a CRT 6 on which CT images reconstructed from projection data are displayed; and a storage device 7 in which programs, data, and X-ray CT images are stored.

The table 10 includes a cradle 12 on which a subject lies down and which comes in or out of the bore of the scanner gantry 20. The cradle 12 is lifted, lowered, or linearly moved by a motor incorporated in the table 10.

The scanner gantry 20 comprises: an X-ray tube 21; an X-ray controller 22; a collimator 23; a multi-detector 24; a data acquisition system (DAS) 25; a rotation controller 26 that rotates the X-ray tube 21 or the like about the body axis of a subject; a controller 29 that transfers control signals or the like to or from the operating console 1 or radiographic table 10; and a slip ring 30.

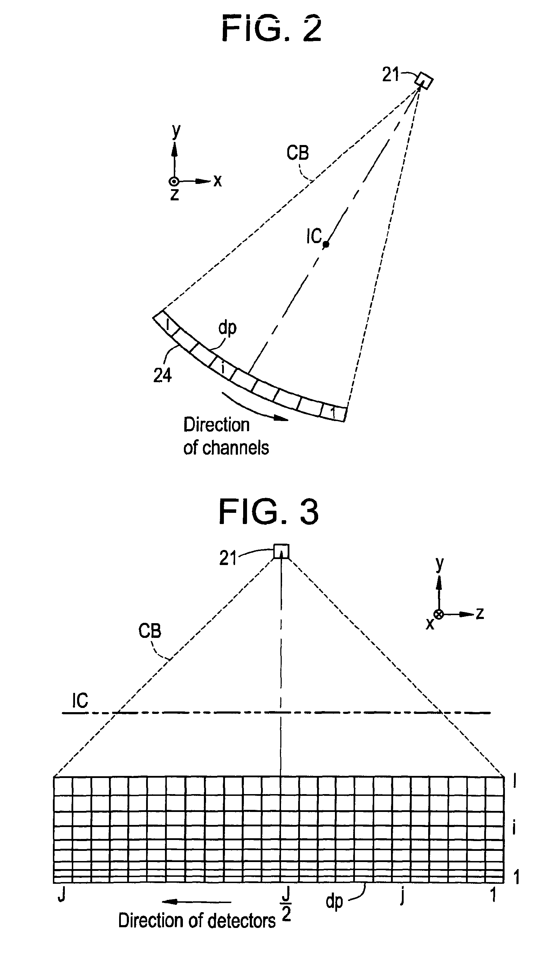

Fig. 2 and Fig. 3 are explanatory diagrams concerning the X-ray tube 21 and multi-detector 24.

The X-ray tube 21 and multi-detector 24 are rotated about a center of rotation IC. Assuming that a vertical direction is a y direction, a horizontal direction is an x direction, and a direction perpendicular to these directions is a z direction, a plane of rotation on which the X-ray tube 21 and multi-detector 24 are rotated is an xy plane. Moreover, a moving direction in which the cradle 12 is moved is the z direction.

The X-ray tube 21 generates an X-ray beam called a conical beam CB. When the center-axis direction of the conical beam CB is parallel to the y direction, the X-ray tube 21 is positioned at a view angle 0°.

The multi-detector 24 includes, for example, 256 arrays of detectors. Each detector array has, for example, 1024 channels.

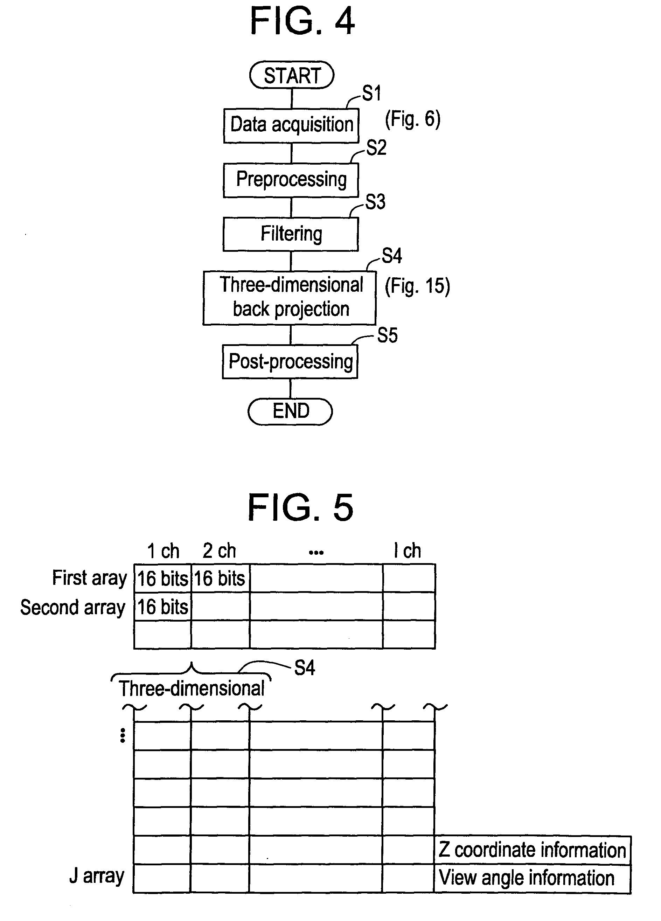

Fig. 4 is a flowchart outlining actions to be performed in the X-ray CT system 100.

At step S1, the X-ray tube 21 and multi-detector 24 are rotated about a subject of radiography, and the cradle 12 is linearly moved. Meanwhile, projection data D0(z,view,j,i) identified with a position z to which the cradle is linear moved, a view angle view, a detector array number j, and a channel number i is acquired. The position z to which the cradle is linear moved is detected by counting the number of position-in-z-axis direction pulses using an encoder. The controller 29 converts the count value into a z-axis coordinate, and appends the z-axis coordinate as z-axis coordinate information to projection data acquired by the DAS 25 via the slip ring 30.

Fig. 5 shows the format for a certain view of projection data having the z-axis coordinate information appended thereto.

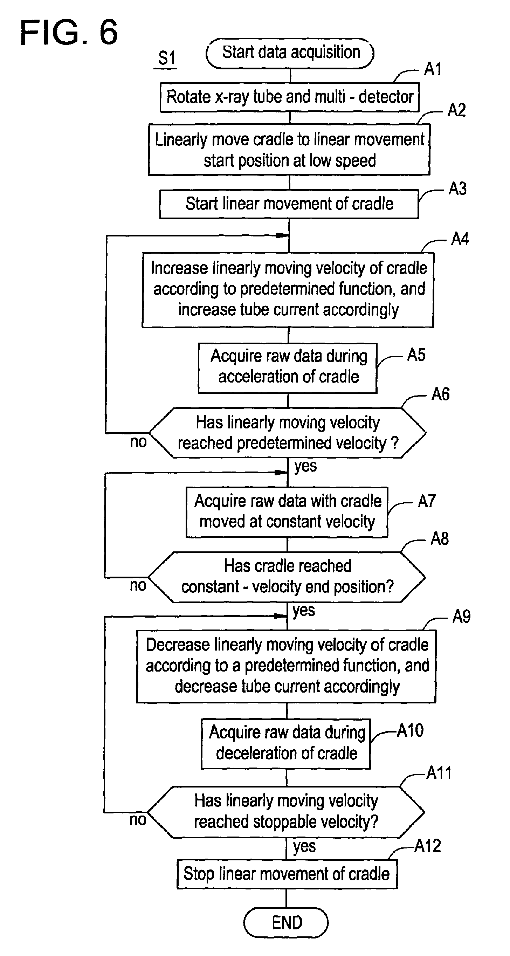

Incidentally, the data acquisition will be described later with reference to Fig. 6 to Fig. 14.

At step S2, the projection data D0(z,view,j,i) is preprocessed (undergoes offset correction, logarithmic correction, exposure correction, and sensitivity correction).

At step S3, the preprocessed projection data D0(z,view,j,i) is filtered. Specifically, the projection data is Fourier-transformed, filtered (assigned to a reconstruction function), and then inverse-Fourier-transformed.

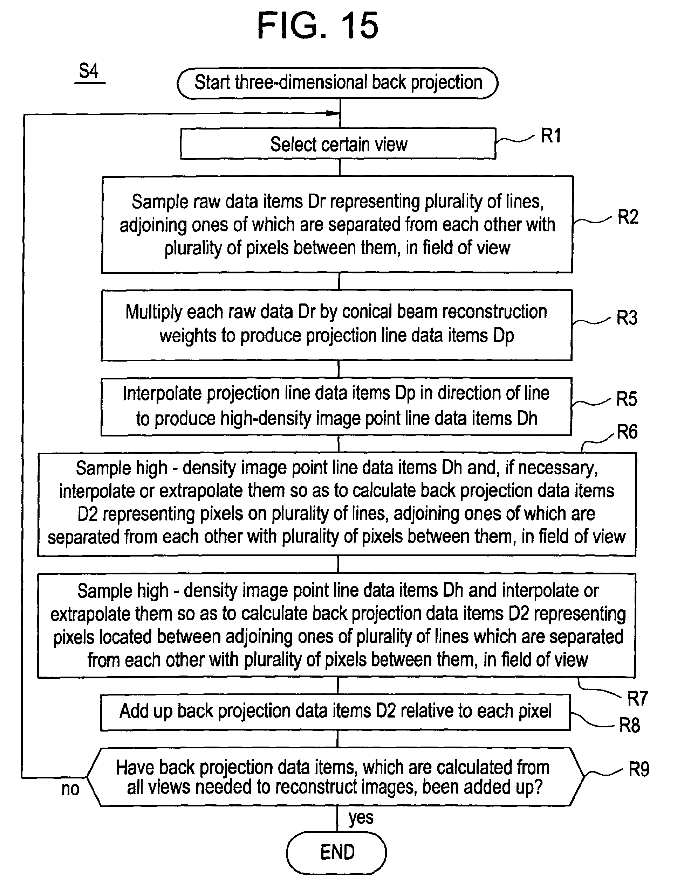

At step S4, three-dimensional back projection is performed on the filtered projection data D0(z,view,j,i) in order to produce back projection data D3(x,y). The three-dimensional back projection will be described with reference to Fig. 15 later.

At step S5, back projection data D3(x,y) is post-processed in order to produce CT images.

Fig. 6 is a flowchart describing data acquisition (step S1 in Fig. 4).

At step A1, the X-ray tube 21 and multi-detector 24 are rotated about a subject of radiography.

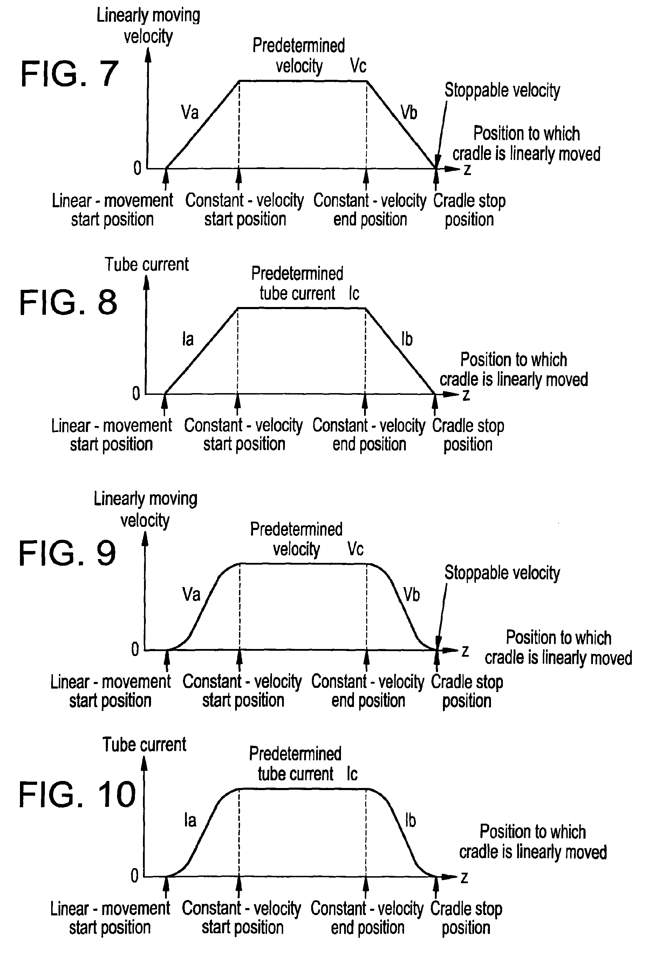

At step A2, the cradle 12 is linearly moved aat low speed to a linear movement start position indicated in Fig. 7 and Fig. 9.

At step A3, the linear movement of the cradle 12 is started.

At step A4, the linearly moving velocity at which the cradle 12 is linearly moved is increased based on a predetermined function, and a tube current is increased accordingly. Fig. 7 and Fig. 8 are graphs of a predetermined function that is linear to a time, while Fig. 9 and Fig. 10 are graphs of a predetermined function that is nonlinear to a time. An X-ray density in the direction of linear movement, that is, an exposure per unit thickness is proportional to a quotient of the tube current by the linearly moving velocity. Consequently, when the tube current is increased with an increase in the linearly moving velocity, the quotient of the tube current by the linearly moving velocity can be held constant. Eventually, the X-ray density in the direction of linear movement can be held constant even during acceleration.

At step A5, projection data D0(z,view,j,i) is acquired during acceleration of the cradle.

At step A6, if the linearly moving velocity of the cradle 12 reaches a predetermined velocity Vc indicated in Fig. 7 and Fig. 9, control is passed to step A7. If the linearly moving velocity does not reach the predetermined velocity Vc, control is returned to step A4. The cradle 12 is further accelerated.

At step A7, projection data D0(z,view,j,i) is acquired with the cradle 12 held at the predetermined linearly moving velocity or at a constant velocity.

At step A8, if the cradle 12 reaches a constant-velocity end position indicated in Fig. 7 and Fig. 9, control is passed to step A9. If the cradle 12 does not reach the constant-velocity end position, control is returned to step A7. Projection data is kept acquired with the cradle 12 moved at the constant velocity.

At step A9, the linearly moving velocity of the cradle 12 is decreased based on a predetermined function, and the tube current is decreased accordingly. Fig. 7 and Fig. 8 are graphs of a predetermined function that is linear to a time, while Fig. 9 and Fig. 10 are graphs of a predetermined function that is nonlinear to a time. An X-ray density in the direction of linear movement, that is, an exposure per unit thickness is proportional to a quotient of the tube current by the linearly moving velocity. Consequently, when the tube current is decreased with a decrease in the linearly moving velocity, the quotient of the tube current by the linearly moving velocity can be held constant. Eventually, the X-ray density in the direction of linear movement can be held constant even during deceleration.

At step A10, projection data D0(z,view,j,i) is acquired during deceleration of the cradle.

At step A11, if the linearly moving velocity of the cradle 12 reaches a stoppable velocity indicated in Fig. 7 and Fig. 9, control is passed to step A12. If the linearly moving velocity of the cradle 12 does not reach the stoppable velocity, control is returned to step A9. The cradle 12 is further decelerated.

At step A12, the linear movement of the cradle 12 is stopped.

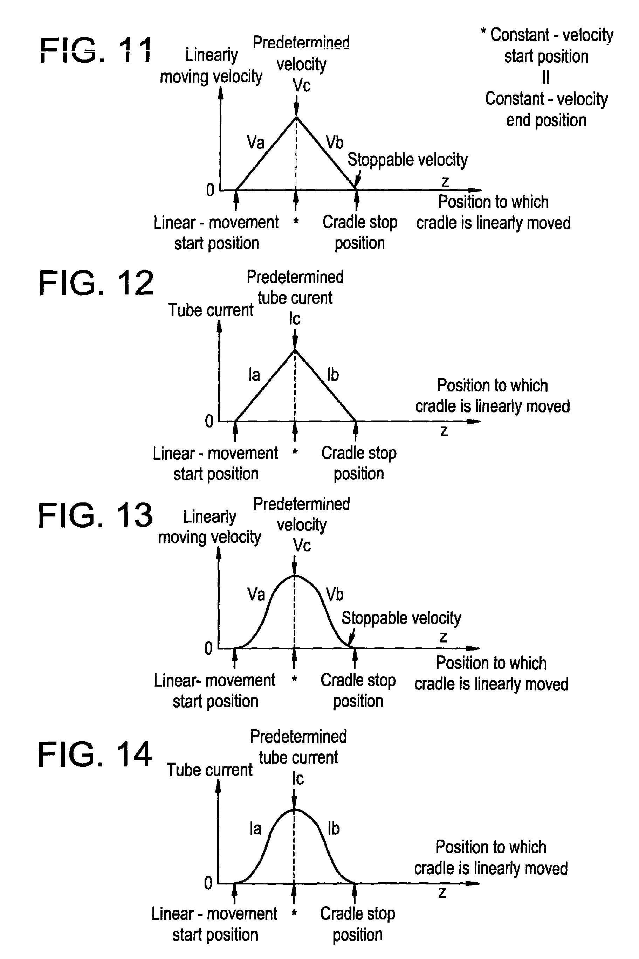

As shown in Fig. 11 to Fig. 14, if the constant-velocity start point and constant-velocity end position are set to the same position, projection data D0(z,view,j,i) can be acquired with the cradle linearly moved the shortest distance.

Fig. 15 is a flowchart describing three-dimensional back projection (step S4 in Fig. 4).

At step R1, one view is selected from all views needed to reconstruct CT images (that is, views acquired by rotating the X-ray tube 360° or views acquired by rotating the X-ray tube 180° plus the angle of a fan beam).

At step R2, projection data items Dr representing a plurality of lines, adjoining ones of which are separated from each other with a plurality of pixels between them, in a field of view are sampled from the selected view composed of projection data items D0(z,view,j,i).

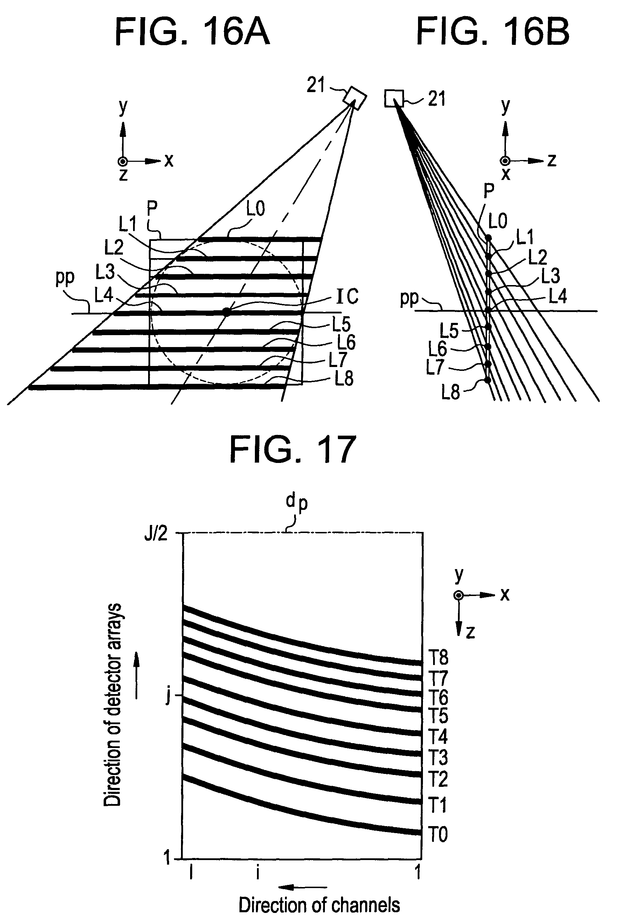

Fig. 16 shows a plurality of parallel lines L0 to L8 in the field of view P.

The number of lines ranges from 1/64 to 1/2 of the largest number of pixels rendered in the field of view in a direction orthogonal to the lines. For example, when the number of pixels in the field of view P corresponds to the product of 512 by 512, the number of lines is 9.

Moreover, when the view angle is equal to or larger than - 45° and smaller than 45° (or a range of view angles centered on this and including others) and is equal to or larger than 135° and smaller than 225° (or a range of view angles centered on this and including others), the x direction is regarded as the direction of lines. Moreover, when the view angle is equal to or larger than 45° and smaller than 135° (or a range of view angles centered on this and including others), and is equal to or larger than 225° and smaller than 315° (or a range of view angles centered on this and including others), the y direction is regarded as the direction of lines.

Moreover, a plane passing the center of rotation IC and parallel to the lines L0 to L8 is regarded as a plane of projection pp.

Fig. 17 shows lines T0 to T8 that are projections of the lines L0 to L8 formed in a direction, in which X-rays are transmitted, on the surface dp of the detector.

The direction in which X-rays are transmitted is determined with the geometric positions of the X-ray tube 21, multi-detector 24, and lines L0 to L8 (including a distance in the z-axis direction from the xy plane, which passes the center in the z-axis direction of the multi-detector 24, to the field of view P, and the positions of the lines L0 to L8 each of which is a set of pixels rendered in the field of view P). Since the position z to which the cradle is linearly moved in order to acquire projection data items D0(z,view,j,i) is known, the direction in which X-rays are transmitted can be accurately detected based on projection data items D0(z,view,j,i) acquired during acceleration or deceleration.

Projection data items that are acquired by the arrays of detectors j on the channels i and that represent the lines T0 to T8 projected on the detector surface dp are sampled and regarded as projection data items Dr representing the lines L0 to L8.

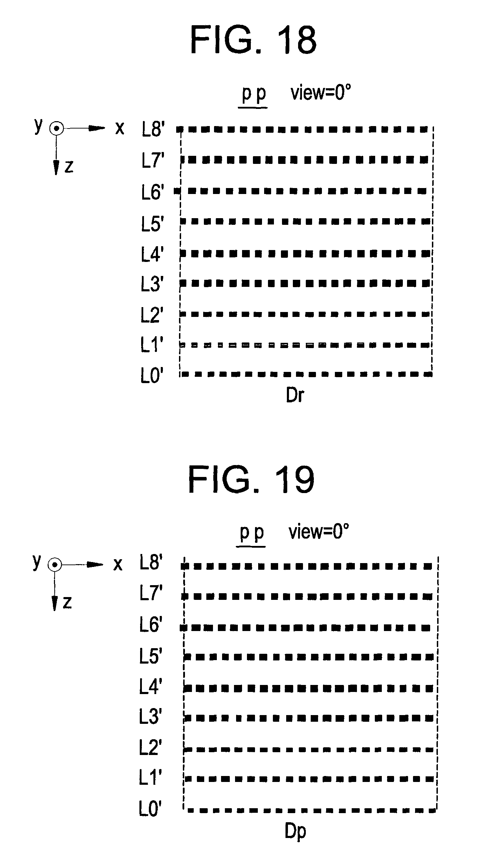

As shown in Fig. 18, lines L0' to L8' are regarded as projections of the lines T0 to T8 formed on the plane of projection pp in the direction in which X-rays are transmitted. The projection data items Dr are developed to represent the lines L0' to L8'.

Referring back to Fig. 15, at step R3, the projection data items Dr representing each of the lines L0' to L8' are multiplied by respective conical beam reconstruction weights in order to produce projection line data items Dp shown in Fig. 19.

Herein, the conical beam reconstruction weight is expressed as (r1/r0)² where r0 denotes a distance from the focal point of the X-ray tube 21 to a position on the multi-detector 24 defined with a detector array number j and channel number i at which projection data Dr is acquired, and r1 denotes a distance from the focal point of the X-ray tube 21 to a pixel in the field of view represented by the projection data Dr.

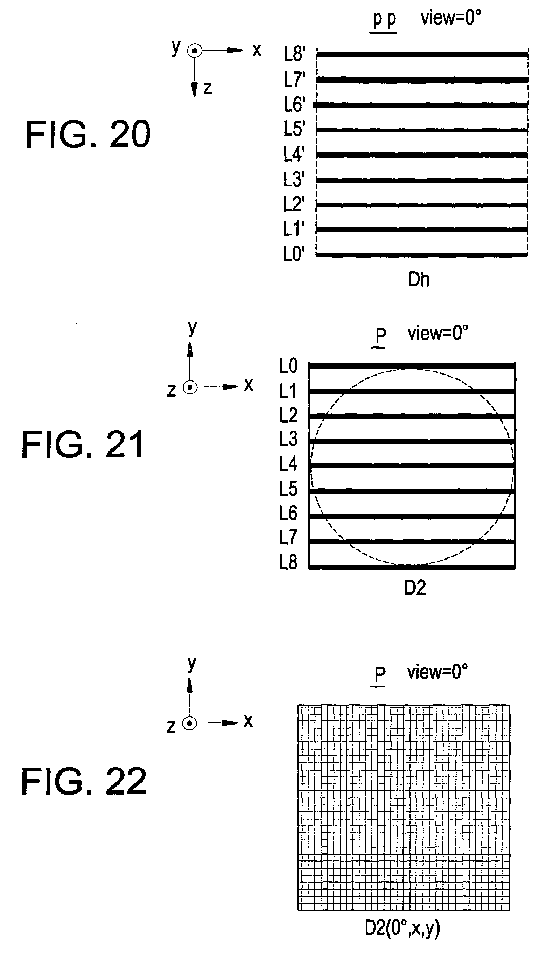

At step R5, the projection line data items Dp are interpolated in the direction of a line in order to produce high-density image point line data items Dh shown in Fig. 20.

The density of the high-density image point line data items Dh is 8 times to 32 times higher than the density equivalent to the largest number of pixels rendered in the direction of a line in the fieid of view. For example, assuming that the data density is 16 times higher, if the number of pixels rendered in the field of view P is the product of 512 by 512, the data density is expressed as 8192 pixels per line.

At step R6, high-density image point line data items Dh are sampled, and, if necessary, interpolated or extrapolated in order to produce, as shown in Fig. 21, back projection data items D2 representing pixels on the lines L0 to L8.

At step R7, high-density image point line data items Dh are sampled, and interpolated or extrapolated in order to produce, as shown in Fig. 22, back projection data items D2 representing pixels on the lines L0 to L8.

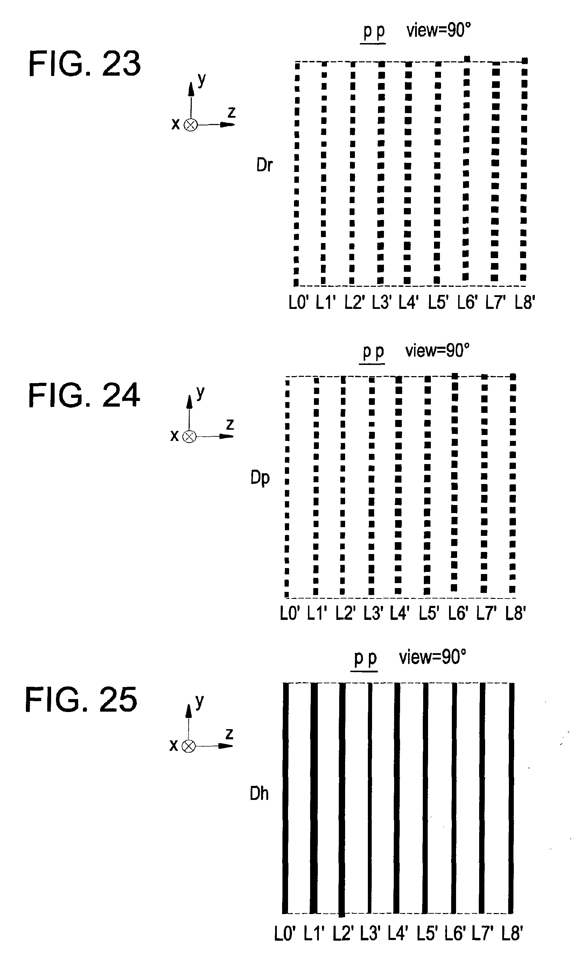

Fig. 18 to Fig. 22 are concerned with a case where the view angle is equal to or larger than - 45° and smaller than 45° (or a range of view angles centered on this and including others), and equal to or larger than 135° and smaller than 225° (or a range of view angles centered on this and including others). Fig. 23 to Fig. 27 are concerned with a case where the view angle is equal to or larger than 45° and smaller than 135° (or a range of view angles centered on this and including others), and equal to or larger than 225° and smaller than 315° (or a range of view angles centered on this and including others).

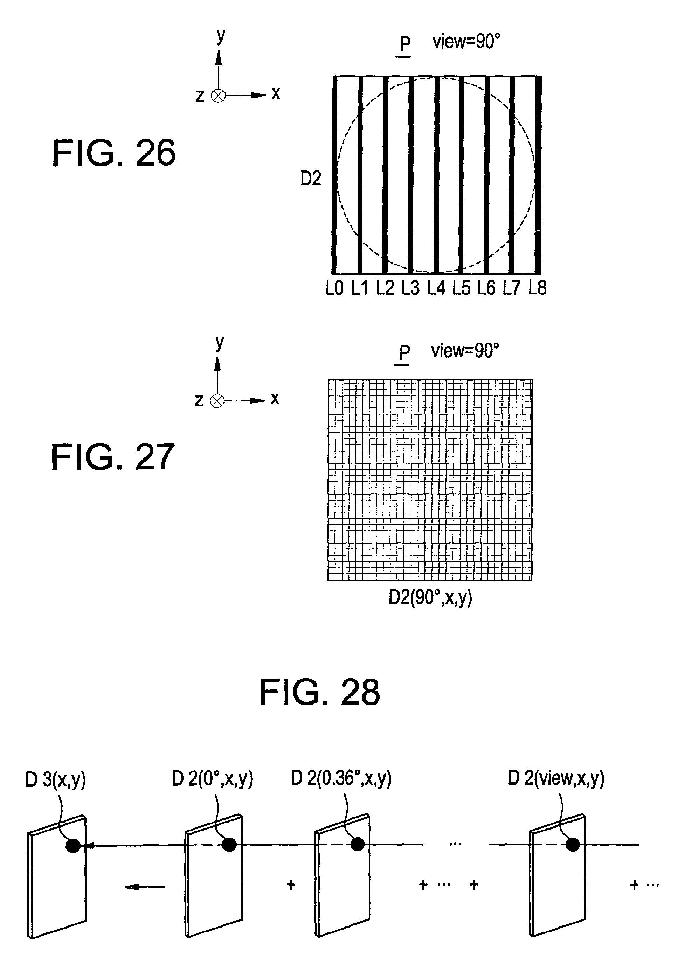

Referring back to Fig. 15, at step R8, as shown in Fig. 28, the back projection data items D2 shown in Fig. 22 or Fig. 27 are added up relative to each pixel.

At step R9, steps R1 to R8 are repeatedly performed on each of all views needed to reconstruct CT images (that is, views acquired by rotating the X-ray tube 360° or 180° plus the angle of a fan beam). This results in back projection data D3(x,y).

According to the X-ray CT system 100 of the first embodiment, projection data can be acquired not only while a linearly moving velocity held constant but also while linear movement is accelerated or decelerated. Acquired projection data is used to reconstruct images. Therefore, a distance linearly moved for acceleration or deceleration out of an overall distance linearly moved can be utilized for image reconstruction.

The image reconstruction technique may be a conventionally known two-dimensional image reconstruction technique or a conventionally known three-dimensional image reconstruction technique including the Feldkamp technique. Furthermore, any of the three-dimensional image reconstruction techniques proposed in Japanese Patent Applications Nos. 2002-066420, 2002-147061, 2002-147231, 2002-235561, 2002-235662, 2002-267833, 2002-322756, and 2002-238947 may be adopted.

### [Second Embodiment]

According to the first embodiment, after views of projection data required for image reconstruction are all acquired at step S1 in Fig. 4, three-dimensional back projection is executed at step S4. In this case, since data acquisition and three-dimensional back projection are performed fully in series with each other, a large time lag is spent until images are produced.

According to the second embodiment, part of three-dimensional back projection is performed concurrently with data acquisition. Consequently, the time lag spent until images are produced can be shortened.

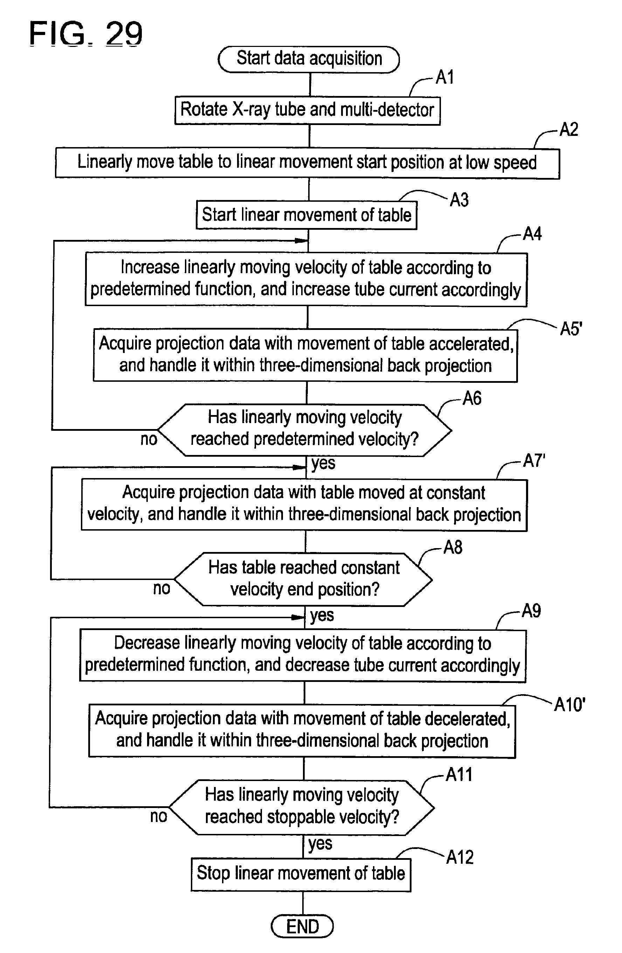

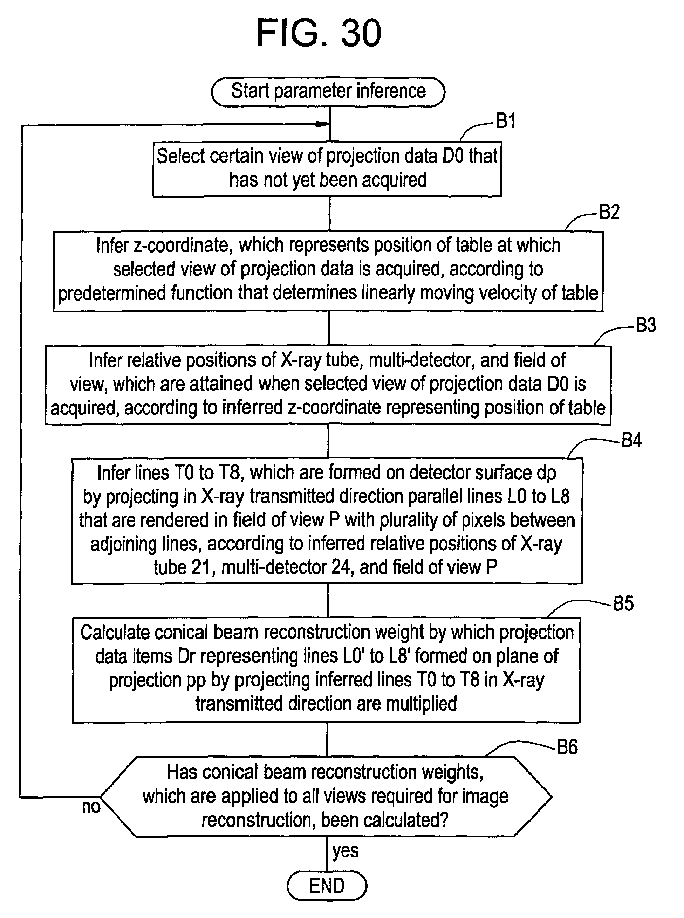

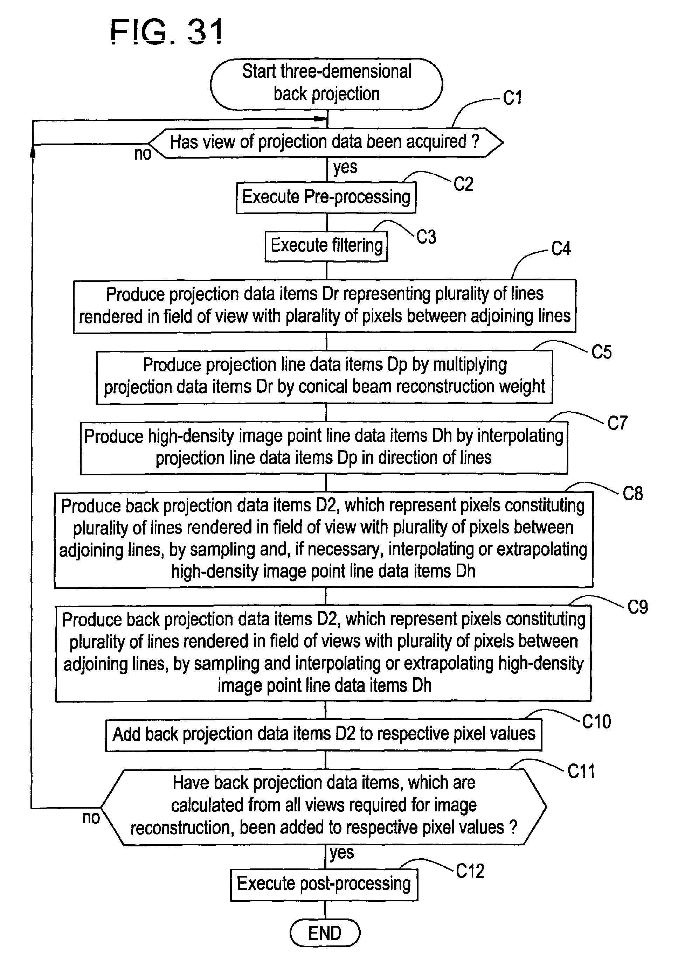

In other words, an X-ray CT system in accordance with the second embodiment concurrently executes data processing described in Fig. 29, parameter inference described in Fig. 30, and three-dimensional back projection described in Fig. 31.

Fig. 29 is a flowchart describing data acquisition executed according to the second embodiment.

The steps described in Fig. 29 are identical to those described in Fig. 6 except steps A5', A7', and A10', so that only the steps A5', A7', and A10' will be described below.

At step A5', projection data D0(z,view,j,i) is acquired with the movement of the table accelerated, and control is concurrently passed to three-dimensional back projection that is under way.

At step A7', projection data D0(z,view,j,i) is acquired with the tab!e moved at a constant velocity, and control is concurrently passed to three-dimensional back projection that is under way.

At step A10', projection data D0(z,view,j,i) is acquired with the movement of the table decelerated, and control is concurrently passed to three-dimensional back projection that is under way.

Fig. 30 is a flowchart describing parameter inference to be executed according to the second embodiment.

At step B1, one view of projection data D0 that has not been acquired is selected.

At step B2, a z-coordinate representing the position of the table 12 at which the selected view of projection data D0 is acquired is inferred based on a predetermined function that determines the linearly moving velocity of the table 12.

At step B3, the relative positions of the X-ray tube 21, multi-detector 24, and field of view P attained when the selected view of projection data D0 is acquired are inferred based on the inferred z-coordinate representing the position of the table 12.

At step B4, lines T0 to T8 to be formed on the detector surface dp by projecting a plurality of parallel lines L0 to L8, which are rendered in the field of view P with a plurality of pixels between adjoining lines, in a direction in which X-rays are transmitted are inferred from the relative positions of the X-ray tube 21, multi-detector 24, and field of view P.

At step B5, a conical beam reconstruction weight by which are multiplied the projection data items Dr representing lines L0' to L8' formed on the plane of projection pp by projecting the inferred lines T0 to T8 in the direction in which X-rays are transmitted is calculated.

At step B6, after the conical beam reconstruction weights to be applied to all views needed for image reconstruction are calculated, processing is completed. If the conical beam reconstruction weight to be applied to any view has not yet been calculated, control is returned to step B1.

Fig. 31 is a flowchart describing three-dimensional back projection to be executed according to the second embodiment.

At step C1, a wait state is established until a view of projection data D0(z,view,j,i) among all views (that is, views acquired with the X-ray tube positioned within 360° or views acquired with the X-ray tube positioned within 180° + the angle of a fan beam) required for reconstructing CT images is selected within data acquisition that is under way (steps A4', A7', and A10'). When the view of projection data D0(z,view,j,i) is selected, control is passed to step C2.

At step C2, the projection data D0(z,view,j,i) selected within data acquisition is pre-processed (subjected to offset correction, logarithmic correction, exposure correction, and sensitivity correction).

At step C3, the pre-processed projection data D0(z,view,j,i) is filtered, or more specifically, Fourier-transformed, filtered (assigned a reconstruction function), and inversely Fourier-transformed.

At step C4, projection data items D0 representing the lines T0 to T8 formed on the detector surface dp by projecting the plurality of parallel lines L1 to L8 rendered in the field of view P with a plurality of pixels between adjoining lines are sampled from the projection data D0(z,view,j,i) selected within data acquisition. The projection data items D0 are developed in order to represent the lines L0' to L8' formed on the plane of projection pp by projecting the lines T0 to T8 in the direction in which X-rays are transmitted, whereby projection data items Dr are produced as shown in Fig. 18.

At this time, if the lines T0 to T8 are inferred in advance within parameter inference that is under way (step B4 in Fig. 30), the projection data items Dr can be produced immediately.

At step C5, the projection data items Dr representing the lines L0' to L8' are multiplied by the conical beam reconstruction weight, whereby projection line data items Dp are produced as shown in Fig. 19.

At this time, if the conical beam reconstruction weight is inferred in advance within parameter inference that is under way (step B5 in Fig. 30), the projection line data items Dp can be produced immediately.

At step C7, the projection line data items Dp are interpolated in the direction of lines, whereby high-density image point line data items Dh are produced as shown in Fig. 20.

At step C8, the high-density image point line data items Dh are sampled and, if necessary, interpolated or extrapolated in order to produce, as shown in Fig. 21, back projection data items D2 representing pixels that constitute lines L0 to L8.

At step C9, the high-density image point line data items Dh are sampled and interpolated or extrapolated in order to produce, as shown in Fig. 22, back projection data items D2 representing the pixels that constitute the lines L0 to L8.

Fig. 18 to Fig. 22 show various kinds of data to be produced on the assumption that the view angle is equal to or larger than -45° and smaller than 45° (or a range of view angles centered on this range and including other neighbor angles) and is equal to or larger than 135° and smaller than 225° (or a range of view angles centered on this range and including other neighbor angles). Fig. 23 to Fig. 27 show equivalent kinds of data to be produced in a case where the view angle is equal to or larger than 45° and smaller than 135° (or a range of view angles centered on this range and including other neighbor angles) and is equal to or larger than 225° and smaller than 315° (or a range of view angles centered on this range and including other neighbor angles).

Referring back to Fig. 31, at step C10, as shown in Fig. 28, the back projection data items D2 shown in Fig. 22 or Fig. 27 are added to respective pixel values.

At step C11, steps C1 to C10 are repeated for all views required for reconstruction of CT images (namely, views acquired with the X-ray tube positioned within 360°, or 180° + the angle of a fan beam), whereby back projection data D3(x,y) is produced. Control is then passed to step C12.

At step C12, the back projection data D3(x,y) is post-processed in order to produce CT images.

According to the X-ray CT system of the second embodiment, not only when a linearly moving velocity is held constant but also when linear movement is accelerated or decelerated, projection data is acquired and utilized for image reconstruction. Therefore, a distance moved linearly for acceleration or deceleration within an overall distance moved linearly can be utilized for image reconstruction.

Furthermore, advantages described below are provided.
(1) Within parameter inference, parameters based on which a conical beam is reconstructed are calculated prior to acquisition of a certain view of projection data D0. Therefore, once the projection data D0 is acquired, it can be handled immediately.
(2) Since data acquisition and three-dimensional back projection are executed concurrently, a time lag spent until images are produced can be reduced.

Incidentally, an image reconstruction method employed may be a Feldkump algorithm that is a generally adopted three-dimensional reconstruction method or any other three-dimensional reconstruction algorithm. Nevertheless, the same advantages as the foregoing ones can be provided.

## Claims

1. An X-ray CT imaging method comprising the steps of: acquiring projection data even when linear movement of a table is accelerated or decelerated during a helical scan (S1, A5, A10, A5', A10'); and utilizing the acquired projection data for image reconstruction (R1-R9, B1-B6, C1-C11).

2. An X-ray CT imaging method comprising the steps of: acquiring projection data even when the linear movement of a table is accelerated or decelerated during a helical scan (S1, A5, A10, A5', A10'); appending coordinate information, which represents the position of said table in a body-axis (hereinafter z-axis) direction during the scan, to each view or several views, or preserving the coordinate information as separate information (S1, B1, B2); and utilizing the acquired projection data for image reconstruction together with the z-coordinate information synchronous with each view or every several views (B3-B6).

3. An X-ray CT imaging method according to Claim 1 or 2, wherein image reconstruction is performed concurrently with acquisition of projection data (S1, A1-A12).

4. An X-ray CT imaging method according to Claim 3, wherein parameters based on which a certain view of projection data is used for image reconstruction are predicted and preserved prior to acquisition of the projection data, or the parameters are predicted during acquisition of the projection data (B4).

5. An X-ray CT imaging method according to Claim 4, wherein: linear movement information representing a change in the position of said table is preserved in advance; a z-coordinate representing the position of said table at which a certain view of projection data is acquired is inferred from the linear movement information prior to acquisition of the projection data (B1-B3); and parameters based on which the projection data is used for image reconstruction are calculated based on the inferred z-coordinate (B4).

6. An X-ray CT system comprising: an X-ray tube (21); an X-ray detector (24); a scanning device (26) that rotates at least one of the X-ray tube (21) and X-ray detector (24) about a subject of radiography, moves both of the X-ray tube (21) and X-ray detector (24) relatively to each other and linearly to the subject of radiography, and acquires projection data even during acceleration or deceleration of linear movement; and an image reconstruction device (3) that produces CT images on the basis of acquired projection data.

7. An X-ray CT system comprising: an X-ray tube (21); an X-ray detector (24); a scanning device (26) for rotating at least one of said X-ray tube and said X-ray detector about a subject of radiography, moving both of them relatively linearly to the subject of radiography, acquiring projection data even when linear movement is accelerated or decelerated, appending coordinate information, which represents the position of a table (12) in a body-axis (hereinafter z-axis) direction during a scan, to each view or several views, or preserving the coordinate information as separate information; and an image reconstruction device (3) for producing CT images on the basis of the acquired projection data and the z-coordinate information synchronous with each view or every several views.

8. An X-ray CT system according to Claim 6 or 7, wherein image reconstruction executed by said image reconstruction device (3) is performed concurrently with acquisition of projection data executed by said scanning device (26).

9. An X-ray CT system according to Claim 8, further comprising a parameter preserving device (7) for predicting and preserving parameters, based on which a certain view of projection data is used for image reconstruction, prior to acquisition of the projection data, or for preserving the parameters while predicting the parameters during acquisition of the projection data.

10. An X-ray CT system according to Claim 9, further comprising: a linear movement information preserving device (7) for preserving in advance linear movement information that represents a change in the position of said table caused by the linear movement; and a parameter inferring device (7) for inferring a z-coordinate, which represents the position of said table at which a certain view of projection data is acquired, from the linear movement information prior to acquisition of the projection data, and calculating parameters, based on which the projection data is used for image reconstruction, according to the inferred z-coordinate.
